# EUROPÄISCHE PATENTANMELDUNG

(11) **EP 2 454 986 A1**
(43) Veröffentlichungstag der Anmeldung: **23.05.2012**
(21) Anmeldenummer: 11187753.6
(22) Anmeldetag: 03.11.2011
(51) Int. Cl.: A61B 1/00, A61B 17/32, A61B 18/14

(54) **Endoskopsystem**

(30) Priorität: 18.11.2010 DE 102010051797
(71) Anmelder: Richard Wolf GmbH, 75438 Knittlingen (DE)
(72) Erfinder: Dolt, Martin, 75438 Knittlingen (DE); Weigel, Martin, 75433 Maulbronn (DE)
(74) Vertreter: Patentanwälte Vollmann & Hemmer

(57) **Zusammenfassung**

Das Endoskopsystem weist eine Abbildungsoptik, die zumindest ein verstellbares optisches Element (75, 75')umfasst, ein relativ zur Abbildungsoptik positionierbares Arbeitsinstrument (10, 10', 10"), einen Wegmesser (50, 50', 50", 55, 55', 55"), der zur Erfassung des vom Arbeitsinstrument (10, 10', 10") bei seiner Positionierung zurückgelegten Weges ausgebildet ist, und eine Steuereinrichtung (60, 60', 60"), die das optische Element (75, 75') in Abhängigkeit vom erfassten Weg verstellt, auf.

## Beschreibung

Die Erfindung betrifft ein Endoskopsystem.

Die Abbildungsoptik medizinischer oder technischer Endoskope ist regelmäßig auf einen an den Einsatzzweck des Endoskops angepassten Arbeitsabstand voreingestellt. Abhängig von den technischen Randbedingungen der Abbildungsoptik (wie beispielsweise ihrer maximalen Abmessungen, der Helligkeit der optischen Abbildung sowie den Vorgaben der Topographie der zu untersuchenden Objekte) ergibt sich eine bestimmte Schärfentiefe, d. h. ein sich entlang der optischen Achse um den vorgesehenen Arbeitsabstand erstreckender, begrenzter Bereich, welcher scharf abgebildet werden kann. Häufig müssen jedoch von einem voreingestellten Arbeitsabstand deutlich abweichend beabstandete und insbesondere bewegte Objekte scharf abgebildet werden. Solche Objekte sind beispielsweise bewegte Arbeitsinstrumente von Endoskopsystemen, beispielsweise medizinische Arbeitsinstrumente zur Untersuchung oder Manipulation von Gewebe.

Hierzu ist es bekannt, den Abstand der Schärfeebene der Abbildungsoptik beispielsweise durch manuelles Nachfokussieren zu ändern oder aber die Abbildungsoptik so stark abzublenden, dass die Schärfentiefe erhöht wird. Solche Nachfokussier- bzw. Abblendverfahren sind auch in automatisierter Weise als Autofokus- bzw. Autoiris-Verfahren bekannt.

Nachteilig ist jedoch, dass die Nachfokussierbarkeit einer Abbildungsoptik oft begrenzt ist. Autofokus-Verfahren sind zudem insbesondere bei der Abbildung strukturarmer Gewebeoberflächen aufgrund plötzlich auftretender Schärfekorrekturen problematisch. Auch gehen schnelle Wechsel des Arbeitsabstandes häufig mit störenden Schärfekorrekturen einher, die den Anwender leicht irritieren. Ferner können zusätzlich ins Bild bewegte Objekte zu Schwankungen der Schärfeebene zwischen diesen Objekten und dem Bildhintergrund führen. Das Abblenden zur Vergrößerung der Schärfentiefe hingegen erfordert häufig eine derart starke Abblendung, dass die damit einhergehende Lichtschwäche der optischen Abbildung die Arbeit mit dem Endoskop stark einschränkt bzw. sogar ausschließt. Bei Abblend- bzw. Autoiris-Verfahren reduziert sich durch starkes Abblenden zudem die optische Auflösung des Endoskopsystems.

Vor diesem Hintergrund ist es Aufgabe der Erfindung, ein Endoskopsystem zu schaffen, mit welchem bewegliche Objekte zuverlässig und anwenderfreundlich scharf abbildbar sind.

Diese Aufgabe wird mit einem Endoskopsystem mit den in Anspruch 1 angegeben Merkmalen gelöst. Bevorzugte Weiterbildungen der Erfindung sind in den Unteransprüchen, der nachfolgenden Beschreibung und den Zeichnungen angegeben.

Das erfindungsgemäße Endoskopsystem weist eine Abbildungsoptik auf, die zumindest ein verstellbares optisches Element umfasst. Ferner weist das Endoskopsystem ein relativ zur Abbildungsoptik positionierbares Arbeitsinstrument, einen Wegmesser, der zur Erfassung des vom Arbeitsinstrument bei seiner Positionierung zurückgelegten Weges ausgebildet ist, und eine Steuereinrichtung, die das optische Element in Abhängigkeit vom erfassten Weg verstellt, auf. Unter der Bewegung des Arbeitsinstruments relativ zur Abbildungsoptik ist dabei eine Bewegung des Arbeitsinstruments relativ zu einem feststehenden Teil der Abbildungsoptik, beispielsweise relativ zu zumindest einem Bildsensor oder einem Okular der Abbildungsoptik, zu verstehen. Bei dem erfindungsgemäßen Endoskopsystem ist die jeweils zu einem Zeitpunkt erwünschte Lage der Schärfeebene der Abbildungsoptik, d. h. derjenigen Ebene, die von der Abbildungsoptik scharf abgebildete Objekte enthält, durch die Positionierung des Arbeitsinstruments relativ zur Abbildungsoptik bestimmt. Beispielsweise ist das Arbeitsinstrument ein medizinisches Instrument zur Untersuchung, Entnahme bzw. zur Manipulation von Gewebe, welches mittels der Abbildungsoptik abgebildet wird, so dass die Führung und/oder die Arbeitsweise des Arbeitsinstrumentes durch visuelle Kontrolle mittels der Abbildungsoptik geprüft bzw. gesteuert wird. Der Wegmesser des erfindungsgemäßen Endoskopsystems erfasst dabei den bei der Positionierung des Arbeitsinstruments durch dieses zurückgelegten Weg, insbesondere der Weg entlang der optischen Achse. Somit erfasst der Wegmesser stets die zur Gewährleistung einer scharfen Abbildung des Arbeitsinstruments erforderlichen Wegänderungen der Schärfeebene der Abbildungsoptik. Das stellbare optische Element der Abbildungsoptik wird nun mittels der Steuereinrichtung in Abhängigkeit vom erfassten Weg derart eingestellt, dass die Schärfeebene der Abbildungsoptik mit dem Abstand des Arbeitsinstruments von der Abbildungsoptik übereinstimmt, d.h. das optische Element wird damit zur Fokussierung bzw. Schärfenachführung der optischen Abbildung des Arbeitsinstruments mittels der Abbildungsoptik eingestellt.

Die Einstellung des einstellbaren optischen Elementes erfolgt bevorzugt derart, dass der Abstand der Schärfeebene der Abbildungsoptik gerade an den Abstand des Arbeitsinstrumentes von der Abbildungsoptik angeglichen wird. Unter Abstand der Schärfeebene im Sinne dieser Erfindung ist dabei stets der Abstand der Schärfeebene vom objektseitigen oder distalen Ende der Abbildungsoptik zu verstehen und stellt somit eine Eigenschaft der Abbildungsoptik selbst dar. In einer alternativen Weiterbildung der Erfindung ist zumindest ein distaler Teil der Abbildungsoptik, insbesondere eine distale Frontlinse, hinsichtlich ihrer Position entlang der optischen Achse einstellbar, beispielsweise in einem starren Endoskop. Der distale Teil der Abbildungsoptik ist in dieser Weiterbildung der Erfindung relativ zu proximalen optischen Elementen der Abbildungsoptik ohne merkliche Änderung der optischen Abbildung verschiebbar. In diesem Falle wird bevorzugt der Abstand des distalen Teils der Abbildungsoptik vom Arbeitsinstrument an den Arbeitsabstand bzw. den Abstand der Schärfeebene der Abbildungsoptik angepasst. In beiden vorgenannten Weiterbildungen wird das einstellbare optische Element also stets derart eingestellt, dass der tatsächliche Abstand des Arbeitsinstrumentes von der Abbildungsoptik und der Abstand der Schärfeebene der Abbildungsoptik einander angeglichen sind.

Auf diese Weise kann das Arbeitsinstrument bei dem erfindungsgemäßen Endoskopsystem mit der Abbildungsoptik stets scharf abgebildet werden. Vorteilhafterweise wird bei dem erfindungsgemäßen Endoskopsystem mit dem zurückgelegten Weg des Arbeitsinstruments ein Betriebsparameter des Endoskopsystems selbst genutzt, und nicht etwa eine passiv aufgenommene, interpretationsbedürftige Information, wie beispielsweise die Bildinformation bei Autofokuslösungen. Auf diese Weise muss sich das erfindungsgemäße Endoskopsystem anders als bei Autofokuslösungen nicht eigens auf die Abbildung kontrastreicher Objekte stützen, sondern erlaubt selbst bei struktur- bzw. kontrastschwachen Objekten oder bei lichtschwachen Verhältnissen eine scharfe Abbildung. Plötzlich einsetzende Schärfekorrekturen treten bei dem erfindungsgemäßen Endoskopsystem nicht auf, so dass der Anwender irritationsfrei arbeiten kann. Ferner ist es bei dem erfindungsgemäßen Endoskopsystem nicht erforderlich, die Schärfentiefe durch Abblenden zu vergrößern. Damit ist die Helligkeit sowie die Auflösung der optischen Abbildung gewährleistet, so dass auch bei lichtschwachen Verhältnissen das Arbeitsinstrument und diesem nahe Objekte lichtstark und zugleich hinreichend scharf abgebildet werden können.

Bevorzugt ist bei dem erfindungsgemäßen Endoskopsystem die Abbildungsoptik mit dem Arbeitsinstrument in einem gemeinsamen Schaft angeordnet. Vorteilhafterweise ist das Arbeitsinstrument im Schaft entlang der Schaftachse positionierbar angeordnet, wobei die Abbildungsoptik zumindest teilweise ortsfest zum Schaft angeordnet ist. Auf diese Weise ist die Positionierung des Arbeitsinstrumentes relativ zur Abbildungsoptik auf eine einzige Richtung festgelegt. Die Position des Arbeitsinstruments ist somit besonders leicht erfassbar. Zweckmäßigerweise ist das Arbeitsinstrument entlang einer Achse verschiebbar, welche mit der optischen Achse der Abbildungsoptik zusammenfällt bzw. parallel zu dieser verläuft und insbesondere geringfügig - verglichen mit typischen Arbeitsabständen - zu der optischen Achse der Abbildungsoptik versetzt ist. Besonders vorteilhaft verläuft die optische Achse der Abbildungsoptik parallel zur Schaftachse, wobei das Arbeitsinstrument entlang der Schaftachse verschiebbar ist.

Bevorzugt ist bei dem erfindungsgemäßen Endoskopsystem die Steuereinrichtung zur Stellung des optischen Elements derart ausgebildet, dass der Abstand der Schärfeebene oder die Position der Abbildungsoptik relativ zu dem erfassten Weg des Arbeitsinstrumentes entlang der optischen Achse geändert wird. Damit ist stets eine scharfe Abbildung des Arbeitsinstruments gewährleistet.

Bevorzugt ist bei dem erfindungsgemäßen Endoskopsystem die Steuereinrichtung zur Verstellung des optischen Elements derart ausgebildet, dass sich die Schärfeebene der Abbildungsoptik mit dem Arbeitsinstrument synchron verschiebt. Alternativ kann die Steuereinrichtung auch die Position eines distalen Teils der Abbildungsoptik, derart verstellen, dass sich dieser distale Teil, beispielsweise eine Frontlinse der Abbildungsoptik, mit dem Arbeitsinstrument entlang einer gemeinsamen Achse synchron mitbewegt.

In einer weiteren bevorzugten Weiterbildung der Erfindung ist das Arbeitsinstrument schräg zur optischen Achse der Abbildungsoptik verschiebbar, d. h. die Abbildungsoptik und das Arbeitsinstrument sind in einem Winkelversatz zueinander angeordnet. Beispielsweise sind die Achse, entlang der das Arbeitsinstrument verschiebbar ist, und die optische Achse der Abbildungsoptik (zumindest während des Betriebs) des Endoskopsystems mit bekanntem Winkel feststehend zueinander angeordnet. Zweckmäßigerweise wird durch die Verstellung des optischen Elements durch die Steuereinrichtung der Abstand der Schärfeebene oder die Position eines distalen Teils der Abbildungsoptik in dieser Weiterbildung stets um den Weg des Arbeitsinstruments entlang der optischen Achse geändert. Das bedeutet, der Abstand der Schärfeebene wird nicht um dasselbe Maß geändert, um welches sich das Arbeitsinstrument verschiebt, vielmehr wird der Winkelversatz zwischen der optischen Achse und der Bewegungsachse des Arbeitsinstrumentes berücksichtigt und der Abstand der Schärfeebene um ein solches Maß verstellt, dass das distale Ende des Arbeitsinstrumentes, d. h. der Arbeitsbereich des Arbeitsinstrumentes, in der Schärfeebene der Abbildungsoptik liegt.

Bevorzugt ist bei dem erfindungsgemäßen Endoskopsystem das zumindest eine verstellbare optische Element ein elektromechanisch und/oder piezoelektrisch verschiebbares und/oder elektrisch veränderbares Element, insbesondere eine Flüssiglinse, deren Brennweite elektrisch veränderbar ist.

Bevorzugt ist oder umfasst das Arbeitsinstrument zumindest eines der nachfolgenden Elemente: eine HF-Elektrode, insbesondere eine HF-Resektions-Elektrode, eine Schneidschlinge, Steinschlinge, Laserfaser, Lithotripsie-Elektrode, Fasszange, ein motorisch betriebenes Arbeitsinstrument, insbesondere ein Fräser oder ein Messtaster. Gerade bei den zuvor genannten Arbeitsinstrumenten handelt es sich um Arbeitsinstrumente, die regelmäßig über eine Abbildungsoptik überwacht werden, so dass hier eine kontinuierlich scharfe Abbildung durch die Abbildungsoptik des Endoskopsystems besonders vorteilhaft ist.

Besonders bevorzugt ist bei dem Endoskopsystem der Wegmesser zur berührungslosen Erfassung des durch das Arbeitsinstrument zurückgelegten Weges ausgebildet. Vorteilhafterweise können dabei Arbeitsinstrument und Teile des Wegmessers des Endoskopschaftsystems unabhängig voneinander gekapselt sein, so dass eine besonders dichte Ausbildung des Endoskopsystems realisierbar ist. Beispielsweise kann ein Teil der Kapselung Teile des Wegmessers voneinander oder vom Arbeitsinstrument trennen, beispielsweise einen Weggeber und einen Wegaufnehmer.

Besonders bevorzugt umfasst bei dem erfindungsgemäßen Endoskopsystem der Wegmesser einen Wegaufnehmer, der fest zur Abbildungsoptik angeordnet ist und einen zum Wegaufnehmer korrespondierenden Weggeber, der fest zum Arbeitsinstrument angeordnet ist. In einer alternativen bevorzugten Weiterbildung umfasst bei dem Endoskopsystem der Wegmesser einen Wegaufnehmer, der fix bzw. fest zum Arbeitsinstrument angeordnet ist und einen zum Wegaufnehmer korrespondierenden Weggeber, der fix bzw. fest zur Abbildungsoptik angeordnet ist. In diesen Weiterbildungen der Erfindung ist die Position des Arbeitsinstrumentes relativ zur Abbildungsoptik und/oder einem weiteren, nicht zugleich mit dem Arbeitsinstrument mitbeweglichen Teil einfach erfassbar.

Zweckmäßigerweise weist der Wegmesser zumindest einen Magnetencoder und/oder Magnetfeldsensoren und/oder eine Tauchspule auf. Beispielsweise ist ein Magnetencoder ein Weggeber des Wegmessers und ein Magnetfeldsensor ein mit dem Magnetencoder korrespondierender Wegaufnehmer. Beispielsweise kann bei dem Wegmesser auch ein Bauteil aus ferromagnetischem Material den Weggeber bilden, welcher bei Relativbewegung von Arbeitsinstrument und Abbildungsoptik mit einer variablen Tiefe, die mit der relativen Positionierung von Arbeitsinstrument und Abbildungsoptik korrespondiert, in eine Tauchspule einragt. Die Tauchspule bildet dabei einen Wegaufnehmer, deren Induktivität durch die Einragtiefe des ferromagnetischen Bauteils geändert wird. Tauchspulen eignen sich dabei besonders zur Erfassung großer Relativbewegungen von Arbeitsinstrument und Abbildungsoptik, beispielsweise bei flexiblen Endoskopsystemen.

Geeigneterweise ist bei dem erfindungsgemäßen Endoskopsystem zumindest eine der nachfolgenden Komponenten vorpositionierbar: Arbeitsinstrument, Wegaufnehmer und/oder Weggeber. Beispielsweise kann bei einem Wechsel des Arbeitsinstruments oder der Abbildungsoptik des Endoskopsystems ein ursprünglich vorgesehener Abstand von Arbeitsinstrument und Abbildungsoptik geändert sein. Beispielsweise kann auch bei einem Wechsel von Wegaufnehmer und/oder -geber eine Änderung solcher Parameter auftreten. In der vorstehend beschriebenen Weiterbildung der Erfindung können dann Arbeitsinstrument, Wegaufnehmer und/oder Weggeber manuell in eine zum Betrieb des Endoskopsystems vorgesehene relative Stellung zueinander vorpositioniert werden. Ausgehend von dieser Ausgangsstellung erfolgt dann die Wegerfassung des Arbeitsinstruments und die korrespondierende Schärfenachführung über das verstellbare optische Element.

In einer alternativen bevorzugten Weiterbildung der Erfindung ist bei dem erfindungsgemäßen Endoskopsystem die Steuereinrichtung ausgebildet zur Stellung des optischen Elements in Abhängigkeit einer Initialpositionierung von Arbeitsinstrument und/oder Wegaufnehmer und/oder Weggeber. In diesem Fall können durch die Steuereinrichtung Abweichungen einer Initialstellung von Arbeitsinstrument und Abbildungsoptik von einer ursprünglich zum Betrieb des Endoskopsystems vorgesehenen Anordnung durch eine korrigierende Anpassung der Steuergrößen zur Stellung des optischen Elementes berücksichtigt werden. Auch können Änderungen in der Anordnung, beispielsweise nach dem Wechsel von Arbeitsinstrument und Abbildungsoptik von Wegaufnehmer relativ zum Weggeber, entsprechend korrigiert werden.

Das erfindungsgemäße Arbeitsinstrument ist zum Einbau in ein erfindungsgemäßes Endoskopsystem wie vorstehend beschrieben ausgebildet und mit einem Wegaufnehmer und/oder Weggeber versehen. Das Arbeitsinstrument weist dabei einen Wegaufnehmer auf, der mit einem Weggeber des erfindungsgemäßen Endoskopsystems korrespondiert, oder aber umfasst einen solchen Weggeber, welcher mit einem Wegaufnehmer des erfindungsgemäßen Endoskopsystems korrespondiert. Das erfindungsgemäße Arbeitsinstrument bildet dabei gerade ein solches Arbeitsinstrument des erfindungsgemäßen Endoskopsystems, welches mit der Abbildungsoptik zur Schärfenachführung wie vorstehend beschrieben zusammenwirkt. Beispielsweise handelt es sich bei dem erfindungsgemäßen Arbeitsinstrument um eine HF-Resektions-Elektrode, eine Steinschlinge, eine Schneidschlinge, ein Arbeitsinstrument mit einer Laserfaser, Lithotripsie-Sonde, Fasszange, HF-Elektrode, ein Arbeitsinstrument mit einer Laserfaser, einen Fräser oder einen Messtaster. Beispielsweise ist das Arbeitsinstrument als Austauschteil für ein erfindungsgemäßes Endoskopsystem vorgesehen.

Nachfolgend ist die Erfindung anhand von in den Zeichnungen dargestellter Ausführungsbeispiele näher erläutert. Es zeigen:
- Fig. 1: ein erfindungsgemäßes Endoskopsystem mit einem starren Schaft in einer Prinzipskizze,
- Fig. 2: ein weiteres erfindungsgemäßes Endoskopsystem mit einem starren Schaft und einen distal angeordneten Bildsensor in einer Prinzipskizze,
- Fig. 3: ein weiteres erfindungsgemäßes Endoskopsystem mit einem flexiblen Schaft in einer Prinzipskizze,
- Fig. 4: die Abbildungsoptik des Endoskopsystems gemäß Fig. 2 in einer Prinzipskizze und
- Fig. 5: die Abbildungsoptik gemäß Fig. 4 bei geänderter Entfernung des Arbeitsinstruments von der Abbildungsoptik, und
- Fix. 6: Abbildungsoptik mit gegenüber dem Arbeitsinstrument winklig stehender optischen Achse.

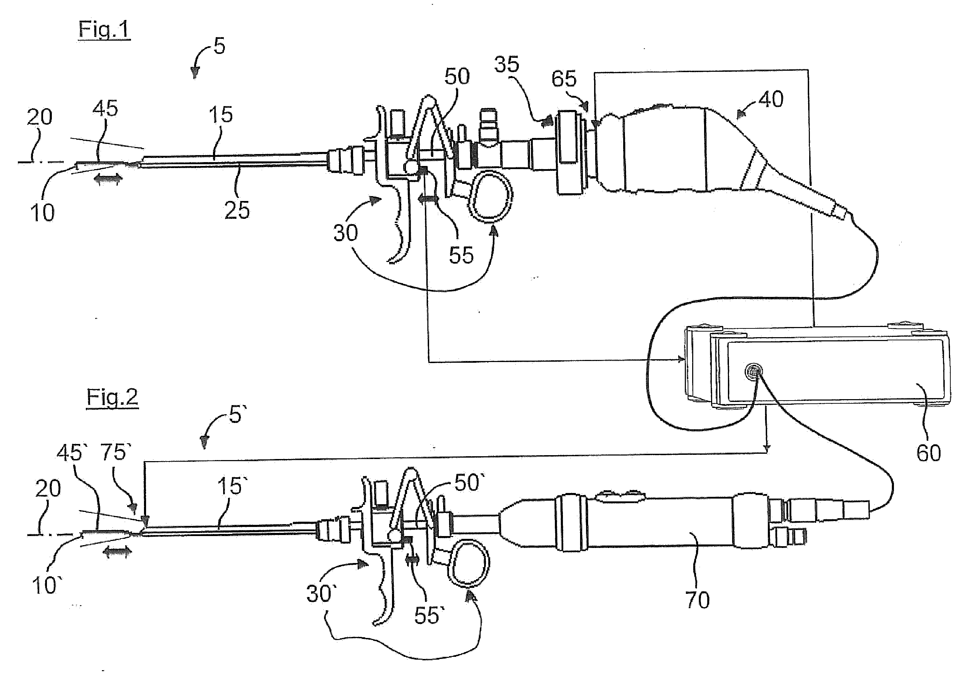

Das in Fig. 1 dargestellte Endoskopsystem 5 ist ein Stablinsenendoskopsystem und weist ein Arbeitsinstrument in Form einer Schlinge 10 auf. Zur visuellen Kontrolle umfasst das Endoskopsystem 5 einen Endoskopschaft 15 mit einer Abbildungsoptik. In diesem Ausführungsbeispiel ist der Endoskopschaft 15 ein urologischer Resektionsschaft und starr ausgebildet. Die Schlinge 10 ist entlang der optischen Achse 20 der Abbildungsoptik des Endoskopschafts 15 beweglich und über einen Instrumentenschaft 25 geführt, welcher sich längs des Endoskopschafts 15 erstreckt und an diesem gehalten ist.

Am proximalen Endbereich des Endoskopschafts 15 ist in üblicher Weise ein Betätigungsmittel 30 zur axialen Bewegung der Schlinge 10 angeordnet. Proximal vom Betätigungsmittel 30 endet der Endoskopschaft 15 in einer Okularkupplung 35. Die Okularkupplung 35 kuppelt den Endoskopschaft 15 an einen abnehmbaren Kamerakopf 40.

Die Bewegung der Schlinge 10 entlang der optischen Achse 20 erfolgt durch die Verschiebung eines im Instrumentenschaft 25 verschiebbaren Aktors 45, der mit der Schlinge 10 und dem Betätigungsmittel 30 bewegungsgekoppelt ist. Die axiale Verschiebung des Aktors 45 wird mittels eines Weggebers 50 erfasst, welcher innerhalb des Endoskopschafts 15 und fix zum Endoskopschaft 15 angeordnet ist. Fix bzw. fest zum Betätigungsmittel 30 und damit auch fix zum Aktor 45 ist am Betätigungsmittel 30 ein Wegaufnehmer 55 vorgesehen, welcher die relativen Verschiebungen des Aktors 45 und damit der Schlinge 10 zum Weggeber 50 erfasst.

Die erfasste Verschiebung der Schlinge 10 entlang der optischen Achse 20 wird mittels einer Steuereinrichtung 60 ausgewertet, mit dem der Wegaufnehmer 55 signalverbunden ist. Abhängig von der axialen Position der Schlinge 10 liefert die Steuereinrichtung 60 eine elektrische Steuergröße, mit welcher ein stellbares optisches Element innerhalb des Kamerakopfes 40, hier eine Flüssiglinse 65 mit elektrisch beeinflussbarer Brennweite, eingestellt und gesteuert wird. Dazu ist die Steuereinrichtung 60 mit einem Steuerkontakt der Flüssiglinse 65 verbunden. Die Flüssiglinse 65 wird dabei derart eingestellt, dass die Abbildung der Schlinge 10 auf einen im Kamerakopf 40 angeordneten Sensor (in der Zeichnung nicht dargestellt) scharf gestellt ist, d. h. der Abstand der Schärfeebene der Abbildungsoptik des Endoskopsystems 5 wird auf die Entfernung der Schlinge 10 vom distalen Ende der Abbildungsoptik im Endoskopschaft 15 eingestellt. Ändert sich der Abstand der Schlinge 10 vom distalen Ende der Abbildungsoptik im Endoskopschaft 15, so wird über den Weggeber 50 und den Wegaufnehmer 55 die Verschiebung der Schlinge 10 erfasst und in der Steuereinrichtung 60 eine neue Steuergröße für die Flüssiglinse 65 ermittelt. Nach der Neuberechnung der elektrischen Steuergröße für die Flüssiglinse 65 übergibt die Steuereinheit die Steuergröße an den Steuerkontakt der Flüssiglinse 65. Infolgedessen wird die Brennweite der Flüssiglinse 65 derart geändert, dass der Abstand der Schärfeebene der Abbildungsoptik des Endoskopsystems 5 an die geänderte Position der Schlinge 10 angepasst wird.

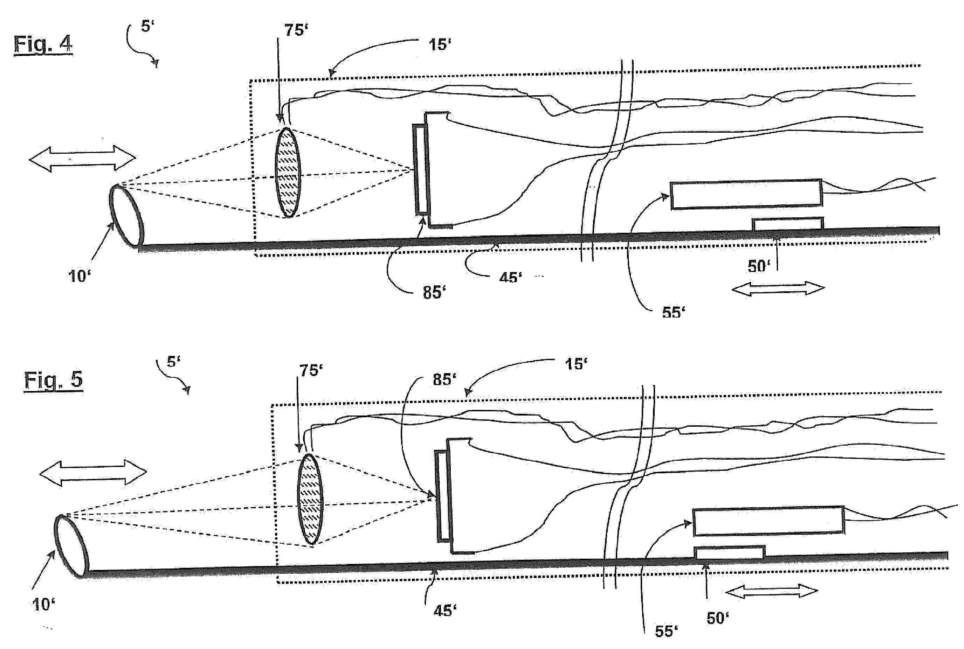

Das in den Fig. 2, 4 und 5 dargestellte Endoskopsystem 5' ist ebenfalls ein Endoskopsystem 5' mit einem starren Endoskopschaft 15'. Das Endoskopsystem 5' ist ein Sensorendoskop, d.h. das Endoskopsystem verfügt über einen Bildsensor 85', der in einem distalen Bereich des Endoskopschafts 15' angeordnet ist (Fig. 4 und 5). Bei dem Endoskopsystem 5' ist das verstellbare optische Element eine Flüssiglinse 75' mit elektrisch beeinflussbarer Brennweite, die eine Frontlinse am distalen Ende des Endoskopschafts 15' bildet.

Auch bei dem Endoskopsystem 5' wird die Verschiebung der Schlinge 10' entlang der optischen Achse 20 über einen Weggeber 50' und einen Wegaufnehmer 55' erfasst. Der Wegaufnehmer 55' befindet sich hierbei fix zur Abbildungsoptik und zum Endoskopschaft 15' innerhalb des Endoskopschafts 15'. Der Weggeber 50' ist dabei fix bzw. fest zum Aktor 45' und zur Schlinge 10', die mit dem Betätigungsmittel 30' bewegungsgekoppelt sind, angeordnet. An den Wegaufnehmer 55' sind mit einer Steuereinrichtung 60' verbundene elektrische Leitungen angebunden, die gemeinsam mit den elektrischen Leitungen des Bildsensors 85' und der Flüssiglinse 75' durch den Endoskopschaft 15' geführt sind. Abhängig von der mittels des Wegaufnehmers 55' erfassten axialen Verschiebung der Schlinge 10' (s. insbesondere Fig. 4 und 5) wird die Flüssiglinse 75' durch die Steuereinrichtung 60' stets derart eingestellt, dass der Abstand der Schärfeebene der Abbildungsoptik dem Abstand der Schlinge 10' vom distalen Ende der Abbildungsoptik, hier der Flüssiglinse 75', entspricht und die Schlinge 10' mittels der Flüssiglinse 75' stets scharf auf den Bildsensor 85' abgebildet wird.

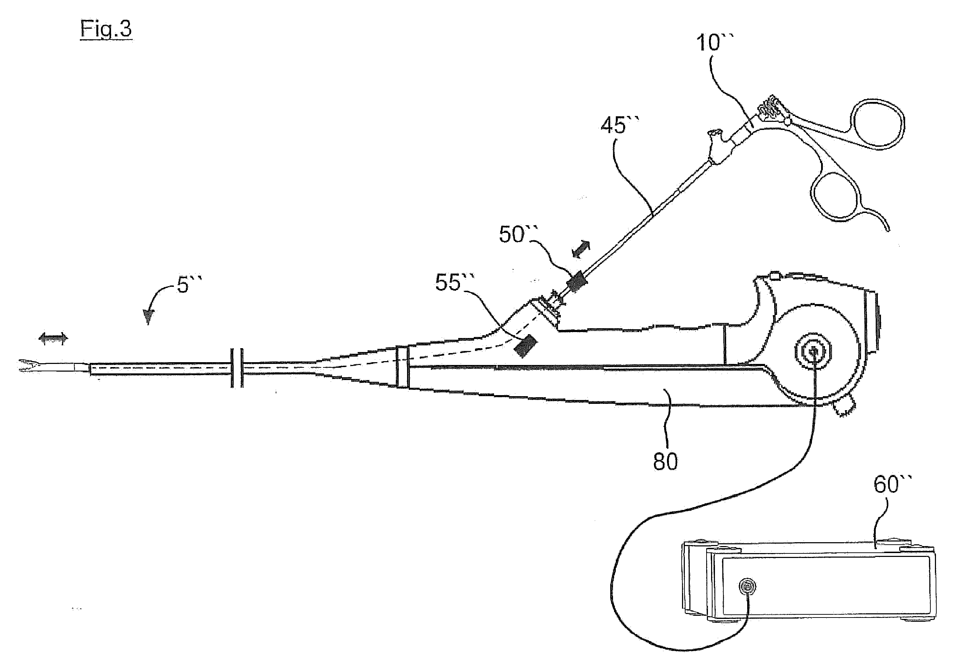

Bei dem in Fig. 3 dargestellten Endoskopsystem 5" sind Arbeitsinstrument und Abbildungsoptik (in der Zeichnung nicht dargestellt) des Endoskopsystems 5" in einem gemeinsamen Endoskopschaft (in der Zeichnung nicht dargestellt) geführt. Bei dem Endoskopsystem 5" wird das Arbeitsinstrument, hier eine Zange 10", mittels eines flexiblen Aktors 45", der im Endoskopschaft geführt ist, entlang des Endoskopschafts verschoben. Die Verschiebung der Zange 10" wird mittels eines fix zum Aktor 45" angeordneten Weggebers 50" und eines fix an einer Handhabe 80" angeordneten Wegaufnehmers 55" erfasst und von einem Wegaufnehmer 55" über ein mit der Verschiebung der Zange 10" korrespondierendes Signal an eine Steuereinrichtung 60" übermittelt. Durch die Steuereinrichtung 60" wird bei der Abbildungsoptik des Endoskopsystems 5" eine Flüssiglinse mit elektrisch beeinflussbarer Brennweite, die das Objektiv der Abbildungsoptik des Endoskopsystems 5" bildet, eingestellt. Die Einstellung erfolgt dabei, wie bei den vorstehenden Ausführungsbeispielen beschrieben, derart, dass die Brennweite der Flüssiglinse soweit geändert wird, dass der Arbeitsabstand der Abbildungsoptik des Endoskopsystems 5" um die Verschiebung der Zange 10" entlang der optischen Achse der Abbildungsoptik des Endoskopsystems 5" korrigiert wird.

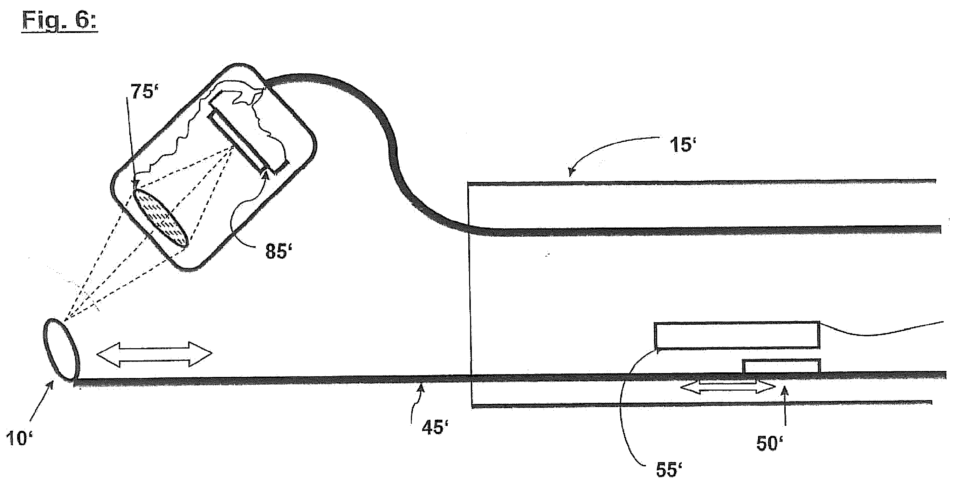

Fig. 6 zeigt eine Ausführungsform, in welcher die Abbildungsoptik, bestehend aus Bildsensor 85' und Linse 75' gewinkelt zur Bewegungsrichtung des Arbeitsinstrumentes bzw. Aktors 45' angeordnet ist. D. h. die Bewegungsrichtung des Aktors bzw. Arbeitsinstrumentes verläuft in einem Winkel zur optischen Achse, die durch die Anordnung von Bildsensor 85' und Objektiv bzw. Linse 75' definiert wird. Durch Anpassung der Form der als Flüssiglinse 75' ausgebildeten Linse kann die Schärfeebene so eingestellt werden, dass die Schlinge 10', welche hier das distale Ende des Arbeitsinstrumentes darstellt, im Bereich der Schärfeebene gelegen ist. Dabei wird die Schärfeebene eben nicht um dasselbe Maß, wie der Aktor 45' bewegt, sondern um ein Maß, welches den Winkel zwischen optischer Achse und Bewegungsachse des Aktors 45' berücksichtigt.

### Bezugszeichenliste

- 5, 5', 5": Endoskopsystem
- 10, 10': Schlinge
- 10": Zange
- 15, 15': Endoskopschaft
- 20: optische Achse
- 25: Instrumentenschaft
- 30, 30': Betätigungsmittel
- 35: Okularkupplung
- 40: Kamerakopf
- 45, 45', 45": Aktor
- 50, 50', 50": Weggeber
- 55, 55', 55": Wegaufnehmer
- 60, 60', 60": Steuereinrichtung
- 65: Flüssiglinse (verstellbares optisches Element)
- 70': Handhabe
- 75': Flüssiglinse
- 80": Handhabe
- 85': Bildsensor

## Patentansprüche

1. Endoskopsystem mit einer Abbildungsoptik, die zumindest ein verstellbares optisches Element (75, 75') umfasst, einem relativ zur Abbildungsoptik positionierbaren Arbeitsinstrument (10, 10', 10"), einem Wegmesser (50, 50', 50", 55, 55', 55"), der zur Erfassung des vom Arbeitsinstrument (10, 10', 10") bei seiner Positionierung zurückgelegten Weges ausgebildet ist, und einer Steuereinrichtung (60, 60', 60"), die das optische Element (75, 75') in Abhängigkeit vom erfassten Weg verstellt.

2. Endoskopsystem nach Anspruch 1, bei welchem die Abbildungsoptik mit dem Arbeitsinstrument (10, 10', 10") in einem gemeinsamen Schaft (15, 15') angeordnet ist.

3. Endoskopsystem nach Anspruch 1 oder 2, bei welchem die Abbildungsoptik in einem Winkelversatz zum Arbeitsinstrument (10, 10',10") angeordnet ist.

4. Endoskopsystem nach einem der vorhergehenden Ansprüche, bei welchem die Steuereinrichtung (60, 60', 60") zur Verstellung des optischen Elements (75, 75') derart ausgebildet ist, dass sich der Abstand der Schärfeebene oder die Position der Abbildungsoptik um den erfassten Weg des Arbeitsinstrumentes (10, 10',10") ändert.

5. Endoskopsystem nach einem der vorhergehenden Ansprüche, bei welchem das zumindest eine verstellbare optische Element (75, 75') ein elektromechanisch und/oder piezoelektrisch verschiebbares und/oder elektrisch veränderbares Element, insbesondere eine Flüssiglinse, ist.

6. Endoskopsystem nach einem der Ansprüche 1 bis 5, bei welchem das Arbeitsinstrument (10, 10', 10") zumindest eines der nachfolgenden Elemente ist oder umfasst: HF-(Resektions-)elektrode, Schneidschlinge, Laserfaser, Lithotripsie-Sonde, Fasszange, motorisch betriebenes Arbeitsinstrument (10, 10', 10"), insbesondere Fräser oder Messtaster.

7. Endoskopsystem nach einem der vorhergehenden Ansprüche, bei welchem der Wegmesser (50, 50', 50"; 55, 55', 55") zur berührungslosen Erfassung des durch das Arbeitsinstrument (10, 10', 10") zurückgelegten Weges ausgebildet ist.

8. Endoskopsystem nach einem der vorhergehenden Ansprüche, bei welchem der Wegmesser (50, 50', 50"; 55, 55', 55") einen Wegaufnehmer (55'), der fix zur Abbildungsoptik angeordnet ist, und einen zum Wegaufnehmer (55') korrespondierenden Weggeber (50'), der fix zum Arbeitsinstrument (10') angeordnet ist, oder einen Wegaufnehmer (55, 55"), der fix zum Arbeitsinstrument (10, 10") angeordnet ist, und einen zum Wegaufnehmer (55, 55") korrespondierenden Weggeber (50, 50"), der fix zur Abbildungsoptik angeordnet ist, umfasst.

9. Endoskopsystem nach einem der vorhergehenden Ansprüche, bei welchem der Wegmesser (50, 50', 50"; 55, 55', 55") einen Magnetencoder und/oder eine Tauchspule aufweist.

10. Endoskopsystem nach einem der vorhergehenden Ansprüche, bei welchem zumindest eine der nachfolgenden Komponenten vorpositionierbar ist: Arbeitsinstrument (10, 10', 10"), Wegaufnehmer (55, 55', 55") und/oder-geber (50, 50', 50").

11. Endoskopsystem nach einem der vorhergehenden Ansprüche, bei welchem die Steuereinrichtung (60, 60', 60") ausgebildet ist zur Stellung des optischen Elements (75') in Abhängigkeit der Vorpositionierung(en) von Arbeitsinstrument (10, 10', 10") und/oder Wegaufnehmer (55, 55', 55") und/oder -geber (50, 50', 50").

12. Arbeitsinstrument (10, 10', 10"), welches zum Einbau in ein Endoskopsystem nach einem der vorhergehenden Ansprüche ausgebildet ist und welches mit einem Wegaufnehmer (55, 55', 55") und/oder Weggeber (50, 50', 50") versehen ist.
